# EUROPÄISCHE PATENTSCHRIFT

(11) **EP 1 077 210 B1**
(45) Veröffentlichungstag und Bekanntmachung des Hinweises auf die Patenterteilung: **28.04.2004**
(21) Anmeldenummer: 00116238.7
(22) Anmeldetag: 07.08.2000
(51) Int. Cl.: C07D 239/30, A01N 43/50

(54) **Verfahren zur Herstellung von 4,6-Dichlor-5-fluorpyrimidin und seine Verwendung als Biocid**
Preparation of 4,6-dichloro-5-fluorpyrimidine and its use as biocidally active agent
Procédé de préparation de 4,6-dichloro-5-fluopyrimidine et son utilisation comme agent biocide

(30) Priorität: 18.08.1999 DE 19939190; 11.10.1999 DE 19948933
(43) Veröffentlichungstag der Anmeldung: 21.02.2001
(73) Patentinhaber: Bayer CropScience AG, 40789 Monheim (DE)
(72) Erfinder: Weintritt, Holger, Dr., 40764 Langenfeld (DE); Lantzsch, Reinhard, Dr., 42115 Wuppertal (DE)

(56) Entgegenhaltungen:
- EP-A- 0 816 345
- WO-A-98/41513
- DE-A- 1 670 780
- DE-A- 19 642 533
- PATENT ABSTRACTS OF JAPAN vol. 011, no. 038 (C-401), 4. Februar 1987 (1987-02-04) & JP 61 205262 A (DAIKIN IND LTD), 11. September 1986 (1986-09-11)

## Beschreibung

Die Erfindung betrifft ein neues Verfahren zur Herstellung von 4,6-Dichlor-5-fluorpyrimidin.

4,6-Dichlor-5-fluorpyrimidin ist ein Zwischenprodukt, das beispielsweise für die Herstellung von Pflanzenschutzmitteln und Farbstoffen eingesetzt werden kann.

Ein Verfahren zur Herstellung von 4,6-Dichlor-5-fluorpyrimidin ist bereits in DE-A1-197 10 609 beschrieben.

In diesem Verfahren wird 4,6-Dihydroxy-5-fluorpyrimidin mit Phosphoroxychlorid und Dimethylanilin als Base versetzt. Zur Aufarbeitung wird zunächst das überschüssige Phosphoroxychlorid abdestilliert und der Rückstand einer Vakuumdestillation unterworfen. Nachteilig bei diesem Verfahren ist, dass die Base in einer relativ großen Menge eingesetzt wird, und nur unter großem Aufwand zurückgewonnen und wieder verwendet werden kann. Außerdem entstehen bei der wäßrigen Aufarbeitung des Destillationsrückstandes große Abwassermengen mit hohem Phosphatgehalt. Die Aufarbeitung ist daher in großtechnischem Maßstab sehr aufwendig.

Die Herstellung von 4,6-Dichlorpyrimidin ist in DE-A1-196 42 533 und DE-A1-195 31 299 beschrieben.

Ein Verfahren zur Herstellung von 4,6-Dihydroxy-5-fluorpyrimidin wird in JP-A2-61 205 262 beschrieben. Bei diesem Verfahren wird als Kondensationsmittel Formamidinhydrochlorid eingesetzt, das ein relativ teures und stark hygroskopisches Kondensationsmittel ist. Daher stellt dieses Verfahren für die großtechnische Produktion keine Alternative dar.

Verfahren zur Herstellung von 4,6-Dihydroxypyrimidin-Derivaten wurden ebenfalls bereits beschrieben (vgl. WO-A1-94/44327, DE-A1-43 23 180, US-5 847 139).

Aufgabe der vorliegenden Erfindung war es nun, ein Verfahren zur Herstellung von 4,6-Dichlor-5-fluorpyrimidin bereitzustellen, das die Darstellung im großtechnischen Maßstab ohne Basenzusatz ermöglicht und bei dem phosphathaltige Abfälle vermieden werden bzw. in geringen Mengen anfallen.

Es wurde nun gefunden, dass man 4,6-Dichlor-5-fluorpyrimidin (I) 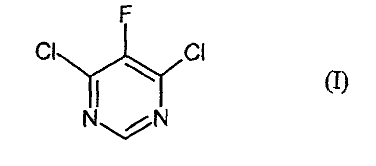 erhält, wenn man
a) Verbindungen der Formel (II), 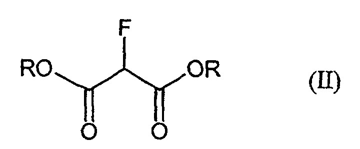 in welcher
   - R: für Methyl oder Ethyl steht,
   mit Formamid in Gegenwart von Alkalimetallalkoholaten, in einem Verdünnungsmittel bei erhöhter Temperatur, gegebenenfalls unter Druck umsetzt, und im Anschluß an die Reaktion das Reaktionsgemisch mit einer Säure ansäuert wobei pro Mol der Verbindung der Formel (II) 2,5 bis 8 Mol Formamid eingesetzt werden und die Zugabe der Verbindung der Formel (II) durch Dosieren innerhalb von 2 bis 4 Stunden erfolgt, und wenn man gegebenenfalls ohne Zwischenisolierung der Verbindungen der Formel (III),
b) das so erhaltene 4,6-Dihydroxy-5-fluorpyrimidin (III) oder dessen Alkalisalz 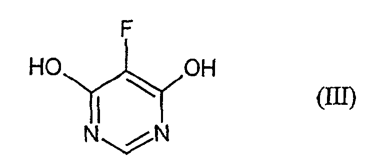 mit Phosphoroxychlorid umsetzt, und anschließend das dabei entstehende Reaktionsgemisch 1 mit Chlor in Gegenwart von Phosphortrichlorid in der Weise umsetzt, dass stets höchstens 99,9 Gew.-% der im Reaktionsgemisch vorhandenen Chlorphosphonsäuren umgesetzt werden, und das dabei entstehende Reaktionsgemisch 2 destillativ aufarbeitet, wobei das entstandene 4,6-Dichlor-5-fluorpyrimidin abgetrennt wird und Phosphoroxychlorid zurückgewonnen wird.

In einer besonders bevorzugten Variante werden die Verbindungen der Formel (II), gegebenenfalls in einem Verdünnungsmittel zu einem Gemisch aus Formamid, Alkalimetallalkoholaten und Verdünnungsmittel gegeben.

Die Vorteile des erfindungsgemäßen Verfahren liegen darin, dass im Verfahrensschritt a) als Kondensationsmittel Formamid eingesetzt wird.

Ein weiterer Vorteil des neuen Verfahrens ist, dass die Chlorierung nach Verfahrensschritt b) ohne Basen durchgeführt werden kann. Außerdem werden große phosphathaltige Abwassermengen vermieden und Phosphoroxychlorid kann zurückgewonnen werden.

Das erfindungsgemäße Verfahren zur Herstellung von 4,6-Dichlor-5-fluorpyrimidin ist daher umweltfreundlicher als die bisher bekannten Verfahren.

Die Ausgangsstoffe der Formel (II) sind bekannte Stoffe und können durch einfache Verfahren hergestellt werden (vgl. DE 42 57 882).

Alle anderen Ausgangsverbindungen sind ebenfalls gängige Handelsprodukte oder können durch einfache Verfahren aus diesen hergestellt werden.

In Formel (II) steht R insbesondere für Ethyl.

Als Verdünnungsmittel zur Durchführung des Verfahrensschrittes a) werden beispielhaft und vorzugsweise Alkohole, insbesondere Alkohole mit 1 bis 4 Kohlenstoffatomen, insbesondere Methanol verwendet.

Als Verdünnungsmittel zur Durchführung des Verfahrensschrittes b) wird vorzugsweise ein Überschuß Phosphoroxychlorid verwendet.

Alkalimetallalkoholate im Sinne der Erfindung sind Kalium- und insbesondere Natriumalkoholate, die sich von Alkoholen mit 1 bis 4 Kohlenstoffatomen ableiten.

Säuren im Sinne der Erfindung sind höher konzentrierte Säuren, insbesondere Mineralsäuren, beispielshaft und vorzugsweise Schwefelsäure, Phosphorsäure, insbesondere Salzsäure.

Die Reaktionstemperaturen können bei der Durchführung des erfindungsgemäßen Verfahrens in einem größeren Bereich variiert werden. Im allgemeinen arbeitet man in einem Temperaturbereich von 50°C bis Rückflußtemperatur der jeweiligen Mischung, insbesondere bei Rückflußtemperatur.

Die Umsetzung von Verbindungen der Formel (II) nach Verfahrensschritt a) erfolgt bei Normaldruck in einem Temperaturbereich von 50°C bis Rückflußtemperatur, vorzugsweise in einem Temperaturbereich von 60°C bis Rückflußtemperatur, unter Druck in einem Temperaturbereich von 60°C bis 120°C, vorzugsweise in einem Temperaturbereich von 80°C bis 110°C.

Die Umsetzung von Verbindungen der Formel (III) nach Verfahrensschritt b) wird vorzugsweise in einem Temperaturbereich von 60°C bis Rückflußtemperatur, insbesondere bei 80°C bis Rückflußtemperatur durchgeführt.

Die Umsetzungen des erfindungsgemäßen Verfahrens erfolgen bei Normaldruck, bei erhöhtem oder bei vermindertem Druck, vorzugsweise bei Normaldruck.

Die Umsetzung von Verbindungen der Formel (II) nach Verfahrensschritt a) erfolgt bei Normaldruck oder bei erhöhtem Druck, insbesondere bei Drücken von 1 bis 4 bar, bevorzugt bei Drücken von 1,5 bis 3 bar.

Die Umsetzung von Verbindungen der Formel (III) nach Verfahrensschritt b) erfolgt bevorzugt bei Normaldruck.

Zur Durchführung des erfindungsgemäßen Verfahrens zur Herstellung der Verbindungen der Formel (III) setzt man in Verfahrensschritt a) pro Mol der Verbindung der Formel (II) im allgemeinen 2,5 bis 8 Mol Formamid, und 3 bis 6 Mol, vorzugsweise 3 bis 4 Mol Alkalimetallalkoholat ein.

Zur Durchführung des erfindungsgemäßen Verfahrens zur Herstellung der Verbindungen der Formel (I) setzt man in Verfahrensschritt b) pro Mol der Verbindung der Formel (III) im allgemeinen 2,5 bis 12 Mol, vorzugsweise 3 bis 8 Mol Phosphoroxychlorid ein.

Zur Durchführung des erfindungsgemäßen Verfahrens zur Herstellung der Verbindungen der Formel (I) werden in Verfahrensschritt b) pro Mol der Verbindungen der Formel (III) im allgemeinen 1,7 bis 2,0 Äquivalente Chlor eingesetzt.

Zur Durchführung des erfindungsgemäßen Verfahrens zur Herstellung der Verbindungen der Formel (I) setzt man in Verfahrensschritt b) pro Mol der Verbindungen der Formel (III) im allgemeinen 1,7 bis 2,0 Äquivalente Phosphortrichlorid ein.

Die Zugabe von Verbindungen der Formel (II) nach Verfahrensschritt a) oder eines Gemisches von Verbindungen der Formel (II) und Formamid, erfolgt insbesondere durch Zutropfen von Verbindungen der Formel (II), die gegebenenfalls in einem Alkohol mit 1 bis 4 Kohlenstoffatomen gelöst sind oder eines Gemisches von Verbindungen der Formel (II) und Formamid, das gegebenenfalls in einem Alkohol mit 1 bis 4 Kohlenstoffatomen gelöst ist, bei Temperaturen von 50°C bis Rückflußtemperatur, insbesondere bei Rückflußtemperatur.

Es ist ein wesentliches Merkmal der Erfindung, dass die Zugabe des Fluormalonesters in Verfahrensschritt a) durch kontinuierliches Dosieren einer Lösung des Fluormalonesters, gegebenenfalls in einem Alkohol mit 1 bis 4 Kohlenstoffatomen zum vorliegenden Reaktionsgemisch erfolgt. Besonders bevorzugt sind Verfahren, in denen die Zugabe durch langsames Dosieren, innerhalb von 2 bis 4 Stunden erfolgt.

In Verfahrensschritt b) setzt man, nachdem die Umsetzung von 4,6-Dihydroxy-5-fluorpyrimidin mit Phosphoroxychlorid erfolgt ist, dem Reaktionsgemisch Phosphortrichlorid und die oben angegebene Menge Chlor so zu, dass stets höchstens 99,9 Gew.-%. der im Reaktionsgemisch vorliegenden Chlorphosphonsäure umgesetzt werden. Dabei erfolgt bevorzugt zuerst die Zugabe von Phosphortrichlorid und anschließend die Zugabe von Chlor.

Die Zugabe von Phosphortrichlorid und Chlor erfolgt bei Temperaturen von 80°C bis Rückflußtemperatur, bevorzugt bei Rückflußtemperatur.

Die Reaktionsdurchführung, Aufarbeitung und Isolierung der Reaktionsprodukte erfolgt nach allgemein üblichen Verfahren (vergleiche auch die Herstellungsbeispiele).

Das erfindungsgemäße Verfahren wird verwendet zur Herstellung von 4,6-Dichlor-5-fluorpyrimidin (I), das ein wichtiges Zwischenprodukt für die Herstellung von Schädlingsbekämpfungsmitteln ist. Nach dem erfindungsgemäßen Verfahren kann man 4,6-Dichlor-5-fluorpyrimidin mit konstant hohen Reinheiten und guten Ausbeuten erhalten. Das neue Verfahren erleichtert daher die Herstellung von bekannten Schädlingsbekämpfungsmitteln.

Die nachfolgenden Beispiele dienen zur Erläuterung der Erfindung. Die Erfindung ist jedoch nicht auf die Beispiele limitiert.

### Herstellungsbeispiele

### Beispiel 1: 4,6-Dihydroxy-5-fluorpyrimidin

30 % Natriummethanolat in Methanol (18.9 g, 0.105 mol) werden zügig mit Formamid (4.7 g, 0.105 mol) versetzt und auf Rückflußtemperatur erhitzt. Anschließend tropft man bei Rückfluß Fluormalonsäureethylester (5.3 g, 28.5 mmol, Gehalt: 95 %) gelöst in Methanol (30 ml) in 3 h zu und rührt weitere 18 h bei Rückflußtemperatur. Die Suspension wird im Vakuum vollständig eingeengt, der Rückstand in Wasser (10 ml) gelöst und mit 30 % HCl auf pH 1 gestellt. Danach wird der ausgefallene Niederschlag abgesaugt und über Nacht bei 100°C im Vakuum getrocknet. Man erhält 4,6-Dihydroxy-5-fluorpyrimidin (3.06 g, Gehalt (HPLC-STD): 74.2 %; 61.4 % d. Th.) als Feststoff.

### Beispiel 2: 4,6-Dihydroxy-5-fluorpyrimidin

30 % Natriummethanolat in Methanol (18.9 g, 0.105 mol) werden zügig mit Formamid (4.7 g, 0.105 mol) versetzt und auf Rückflußtemperatur erhitzt. Anschließend tropft man bei Rückfluß Fluormalonsäureethylester (5.3 g, 28.5 mmol, Gehalt: 95 %) gelöst in Methanol (10 ml) in 3 h zu, überführt das Reaktionsgemisch mit Methanol (20 ml) in einen Autoklav und rührt weitere 3 h bei 100°C (ca. 2.5 bis 3 bar). Die Suspension wird im Vakuum vollständig eingeengt, der Rückstand in Wasser (10 ml) gelöst und mit 30 % HCl auf pH 1 gestellt. Danach wird der ausgefallene Niederschlag abgesaugt und über Nacht bei 100°C im Vakuum getrocknet. Man erhält 4,6-Dihydroxy-5-fluorpyrimidin (3.01 g, Gehalt (HPLC-STD): 79.3 %; 64.4 % d. Th.) als Feststoff.

### Beispiel 3: 4,6-Dihydroxy-5-fluorpyrimidin

30 % Natriummethanolat in Methanol (63.0 g, 0.35 mol) werden zügig mit Formamid (22.5 g, 0.5 mol) versetzt und auf Rückflußtemperatur erhitzt. Anschließend tropft man bei Rückfluß Fluormalonsäureethylester (17.8 g, 95.0 mmol, Gehalt: ca. 95 %) in 225 min zu und rührt weitere 3 h bei Rückflußtemperatur. Die Suspension wird im Vakuum vollständig eingeengt, der Rückstand in Wasser (65 ml) gelöst und mit 30 % HCl auf pH 1 gestellt. Danach wird der ausgefallene Niederschlag abgesaugt und über Nacht bei 100°C im Vakuum getrocknet. Man erhält 4,6-Dihydroxy-5-fluorpyrimidin (11.74 g, Gehalt (HPLC-STD): 82.5 %; 78.4 % d. Th.) als Feststoff.

### Beispiel 4 4,6-Dihydroxy-5-fluorpyrimidin

30 % Natriummethanolat in Methanol (63.0 g, 0.35 mol) werden zügig mit Formamid (33.75 g, 0.75 mol) versetzt und auf Rückflußtemperatur erhitzt. Anschließend tropft man bei Rückfluß Fluormalonsäureethylester (17.8 g, 95.0 mmol, Gehalt: ca. 95 %) in 200 min zu und rührt weitere 3 h bei Rückflußtemperatur. Die Suspension wird im Vakuum vollständig eingeengt, der Rückstand in Wasser (65 ml) gelöst und mit 30 % HCl auf pH 1 gestellt. Danach wird der ausgefallene Niederschlag abgesaugt und über Nacht bei 100°C im Vakuum getrocknet. Man erhält 4,6-Dihydroxy-5-fluorpyrimidin (12.1 g, Gehalt (HPLC-STD): 90.2 %, 88.3 % d. Th.) als Feststoff.

### Beispiel 5: 4,6-Dichlor-5-fluorpyrimidin

51,8 g (0,375 mol, Gehalt: 94,2 %) 4,6-Dihydroxy-5-fluorpyrimidin werden in 287,4 g (1,875 mol) POCl₃ suspendiert, auf Rückflußtemperatur erhitzt und 4 Stunden bei dieser Temperatur gerührt. Anschließend gibt man unter Rückfluß 102 g (0,74 mol) Phosphortrichlorid zur Reaktionsmischung, leitet daraufhin 52,6 g (0,74 mol) Cl₂ ein und rührt weitere 4 h bei Rückflußtemperatur. Nach erfolgter Umsetzung wird Phosphoroxychlorid zusammen mit dem Produkt unter vermindertem Druck vom Rückstand (8,9 g) abgetrennt, und das so erhaltene Destillat (445,6 g) einer Feindestillation unterworfen. Man erhält 384.3 g POCl₃ (95,8 % d.Th., Gehalt: >99,5 %) und 52,5 g 4,6-Dichlor-5-fluorpyrimidin (81,6 % d.Th., Gehalt: 97,3 %) als farblose Flüssigkeiten.

## Patentansprüche

1. Verfahren zur Herstellung von 4,6-Dichlor-5-fluorpyrimidin (I), 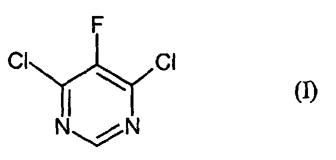 **dadurch gekennzeichnet, dass** man
a) Verbindungen der Formel (II), 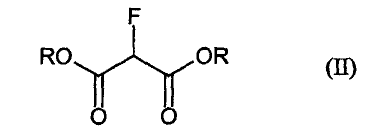 in welcher
R für Methyl oder Ethyl steht,
mit Formamid in Gegenwart von Alkalimetallalkoholaten, in einem Verdünnungsmittel bei erhöhter Temperatur, gegebenenfalls unter Druck umsetzt, und im Anschluß an die Reaktion das Reaktionsgemisch mit einer Säure ansäuert,
wobei pro Mol der Verbindung der Formel (II) 2,5 bis 8 Mol Formamid eingesetzt werden und die Zugabe der Verbindung der Formel (II) durch Dosieren innerhalb von 2 bis 4 Stunden erfolgt,
und
b) das so erhaltene 4,6-Dihydroxy-5-fluorpyrimidin (III) 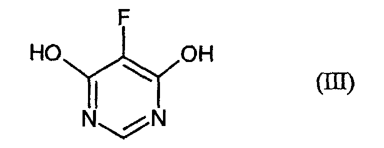 mit Phosphoroxychlorid umsetzt, und anschließend das dabei entstehende Reaktionsgemisch 1 mit Chlor in Gegenwart von Phosphortrichlorid in der Weise umsetzt, dass stets höchstens 99,9 Gew.-% der im Reaktionsgemisch vorhandenen Chlorphosphonsäure umgesetzt werden, und das dabei entstehende Reaktionsgemisch 2 destillativ aufarbeitet, wobei das entstandene 4,6-Dichlor-5-fluorpyrimidin abgetrennt und Phosphoroxychlorid zurückgewonnen wird.

2. Verfahren gemäß Anspruch 1, **dadurch gekennzeichnet, dass** das nach Verfahrensschritt a) erhaltene Produkt nicht zwischenisoliert wird.

3. Verfahren gemäß mindestens einem der Ansprüche 1 bis 2, **dadurch gekennzeichnet, dass** man in Verfahrensschritt a) Verbindungen der Formel (II) zu einem Gemisch aus Formamid und Alkalimetallalkoholat, gegebenenfalls in einem Verdünnungsmittel gibt.

4. Verfahren gemäß mindestens einem der Ansprüche 1 bis 3, **dadurch gekennzeichnet, dass** man in Verfahrensschritt b) pro Mol der Verbindung der Formel (III) 2,5 bis 12 mol Phosphoroxychlorid einsetzt.

5. Verfahren gemäß mindestens einem der Ansprüche 1 bis 4, **dadurch gekennzeichnet, dass** man in Verfahrensschritt b) pro Mol der Verbindung der Formel (III) 1,7 bis max. 2,0 Äquivalente Chlor einsetzt.

6. Verfahren gemäß mindestens einem der Ansprüche 1 bis 5, **dadurch gekennzeichnet, dass** man in Verfahrensschritt b) pro Mol der Verbindung der Formel (III) 1,7 bis max. 2,0 Äquivalente Phosphortrichlorid einsetzt.

## Claims

1. Process for preparing 4,6-dichloro-5-fluoro-pyrimidine (I), 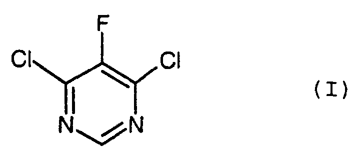 **characterized in that**
a) compounds of the formula (II), 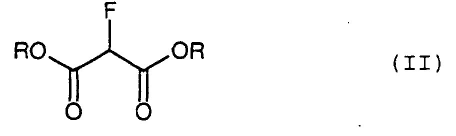 in which
R represents methyl or ethyl
are reacted with formamide in the presence of alkali metal alkoxides in a diluent at elevated temperature, if appropriate under pressure, and the reaction mixture is acidified with an acid after the reaction,
where from 2.5 to 8 mol of formamide are employed per mole of the compound of the formula (II) and the compound of the formula (II) is added by metered addition over a period of from 2 to 4 hours,
and
b) the resulting 4,6-dihydroxy-5-fluoropyrimidine (III) 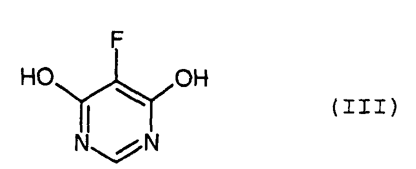 is reacted with phosphorus oxychloride, and the resulting reaction mixture 1 is subsequently reacted with chlorine in the presence of phosphorus trichloride such that in each case at most 99.9% by weight of the chlorophosphonic acid which is present in the reaction mixture are converted, and the resulting reaction mixture 2 is worked up distillatively, whereby the 4,6-dichloro-5-fluoropyrimidine formed is separated off and the phosphorus oxychloride is recovered.

2. Process according to Claim 1, **characterized in that** the product obtained from process step a) is not intermediately stored.

3. Process according to at least one of the claims 1 to 2, **characterized in that**, in process step a), compounds of the formula (II) are added to a mixture of formamide and alkali metal alkoxide, if appropriate in a diluent.

4. Process according to at least one of Claims 1 to 3, **characterized in that**, in process step b), from 2.5 to 12 mol of phosphorus oxychloride are employed per mole of the compound of the formula (III).

5. Process according to at least one of claims 1 to 4, **characterized in that**, in process step b), from 1.7 to at most 2.0 equivalents of chlorine are employed per mole of the compound of the formula (III).

6. Process according to at least one of Claims 1 to 5, **characterized in that**, in process step b), from 1.7 to at most 2.0 equivalents of phosphorus trichloride are employed per mole of the compound of the formula (III).

## Revendications

1. Procédé de production de 4,6-dichloro-5-fluoro-pyrimidine (I), 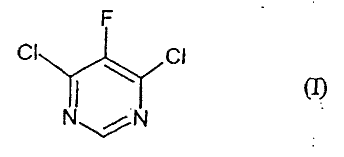 **caractérisé en ce que** :
a) on fait réagir des composés de formule (II), 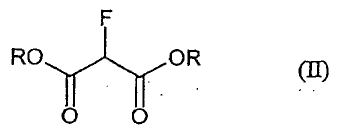 dans laquelle
R est un reste méthyle ou éthyle, avec le formamide en présence d'alkoholates de métaux alcalins, dans un diluant, à température élevée, éventuellement sous pression, puis on acidifie le mélange réactionnel avec un acide à la suite de la réaction,
en utilisant par mol du composé de formule (II) 2,5 à 8 mols de formamide et en effectuant l'addition du composé de formule (II) par dosage dans un intervalle de 2 à 4 heures.
et
b) on fait réagir la 4,6-dihydroxy-5-fluorpyrimidine (III) 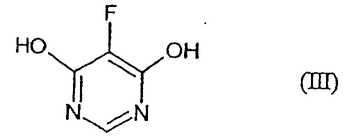 avec l'oxychlorure de phosphore, puis on fait réagir le mélange réactionnel 1 ainsi produit avec du chlore en présence de trichlorure de phosphore de façon telle qu'il y ait toujours au maximum une réaction de 99,9% en poids de l'acide chlorophosphonique present dans le mélange réactionnel, et on traite par distillation le mélange réactionnel 2 ainsi produit, en séparant alors la 4,6-dichloro-5-fluoropyrimidine formée et en recyclant l'oxychlorure de phosphore.

2. Procédé suivant la revendication 1, **caractérisé en ce qu'**il n'y a pas isolement intermédiaire du produit obtenu selon l'étape a).

3. Procédé suivant au moins l'une des revendications 1 et 2, **caractérisé en ce qu'**on ajoute, dans son étape a) , des composés de formule (II) à un mélange de formamide et d'alkoholate de, métal alcalin, éventuellement dans un diluant.

4. Procédé suivant au moins l'une des revendications 1 à 3, **caractérisé en ce qu'**on utilise dans son étape b) 2,5 à 12 mols d'oxychlorure de phosphore par mol de composé de formule (III).

5. Procédé suivant au moins l'une des revendications 1 à 4, **caractérisé en ce qu'**on utilise dans son étape b) 1,7 à, au maximum, 2,0 équivalents de chlore par mol du composé de formule (III).

6. Procédé suivant au moins l'une des revendications 1 à 5, **caractérisé en ce qu'**on utilise dans son étape b) 1,7 à un maximum de 2,0 équivalents de trichlorure de phosphore par mol du composé de formule (III).
